# EUROPEAN PATENT SPECIFICATION

(11) **EP 1 217 076 B1**
(45) Date of publication and mention of the grant of the patent: **14.06.2006**
(21) Application number: 01130740.2
(22) Date of filing: 21.12.2001
(51) Int. Cl.: C12P 13/08, C12P 21/00

(54) **Method of producing a target substance by fermentation**
Verfahren zur Herstellung einer Zielsubstanz durch Fermentation
Procédé pour la production fermentative d'une substance désirée

(30) Priority: 22.12.2000 JP 2000390758
(43) Date of publication of application: 26.06.2002
(73) Proprietor: Ajinomoto Co., Inc., Tokyo 104 (JP)
(72) Inventor: Imaizumi, Akira, c/o Ajinomoto Co., Inc., Kawasaki-ku, Kawasaki-shi, Kanagawa (JP); Usuda, Yoshihiro, c/o Ajinomoto Co., Inc., Kawasaki-ku, Kawasaki-shi, Kanagawa (JP); Sugimoto, Shinichi, c/o Ajinomoto Co., Inc., Kawasaki-ku, Kawasaki-shi, Kanagawa (JP)
(74) Representative: Strehl Schübel-Hopf & Partner

(56) References cited:
- CHOU CHIH-HSIUNG ET AL: "Genetic manipulation of stationary-phase genes to enhance recombinant protein production in Escherichia coli." BIOTECHNOLOGY AND BIOENGINEERING, vol. 50, no. 6, 1996, pages 636-642, XP002192962 ISSN: 0006-3592
- WADA AKIR ET AL: "Growth phase-coupled changes of the ribosome profile in natural isolates and laboratory strains of Escherichia coli." JOURNAL OF BACTERIOLOGY, vol. 182, no. 10, May 2000 (2000-05), pages 2893-2899, XP002192963 ISSN: 0021-9193

### Sequence Listing

<110> Ajinomoto Co., Inc.
<120> Method of Producing Target Substance by Fermentation
<130> EPA-53796
<140>
   <141> 2001-12-21
<150> JP 2000-390758
   <151> 2000-12-22
<160> 4
<170> PatentIn version 3.0
   <210> 1
   <211> 22
   <212> DNA
   <213> Artificial Sequence
<220>
   <223> Description of Artificial Sequence: primer for PCR
<400> 1
   gaacaggcaa ccagtacgct tt
   <210> 2
   <211> 42
   <212> DNA
   <213> Artificial Sequence
<220>
   <223> Description of Artificial Sequence: primer for PCR
<400> 2
   cccatccact aaacaccgtc agttgatgtg cccgttccag gc
   <210> 3
   <211> 29
   <212> DNA
   <213> Artificial Sequence
<220>
   <223> Description of Artificial Sequence: primer for PCR
<400> 3
   tgacggtgtt tagtggatgg gcaaaggtca caatggctg
   <210> 4
   <211> 24
   <212> DNA
   <213> Artificial Sequence
<220>
   <223> Description of Artificial Sequence: primer for PCR
<400> 4
   tacagtgaag ttgatggaga tagt

## Description

### Technical Field

The present invention relates to the fermentation industry. More specifically, the present invention relates to a method for efficiently producing an L-amino acid by fermentation utilizing a microorganism.

### Background Art

In *Escherichia coli*, the protein translation activity decreases during transition from the growth phase to the stationary phase. This phenomenon is considered to be attributable to dimerization of intracellular ribosomes. As a protein involved in this ribosome dimerization, the RMF protein, which is a small protein consisting of 55 amino acid residues and has a molecular weight of 6.5 kDa, has been found (Wada *et al., Proc. Natl. Acad. Sci. USA, 87*, pp.2657-2661, 1990).

The *rmf* gene coding for the RMF protein has been confirmed to exist at a position of 21.8 minutes on *Escherichia coli* chromosome. It has been revealed that expression of the *rmf* gene is suppressed in the logarithmic growth phase, in which cells grow rapidly, that the expression is induced during transition to the stationary phase or during a slow growth rate period and that the expression of this gene is not regulated by the oS factor, which is known to be responsible for stationary phase-specific gene expression (Yamagishi *et*. *al.*, *The EMBO Journal., 12,* pp.625-630, 1993). Further, it has been reported that disruption of the *rmf* gene results in that ribosome dimers disappears also in the stationary phase as a phenotype and survival rate in the stationary phase is lowered (Yamagishi et al., The EMBO Journal, 12, pp.625-630, 1993). Further, it has also been reported that a strain in which this gene is overexpressed cannot grow (Wada *et al.*, *Biochem. Biophys. Res. Comm.*, *214*, pp.410-417, 1995), and it is strongly suggested that this gene is a factor involved in growth of *Escherichia coli* and their survival in the stationary phase.

Chou Chih-Hsiung et al.: "Genetic manipulation of stationary-phase genes to enhance recombinant protein production in Escherichia coli" (Biotechnology and Bioengineering, vol. 50, no. 6, 1996, pages 636-624, XP002192962 ISSN: 0006-3592) discloses the manipulation of the rmf gene to enhance recombinant protein production in Escherichia coli.

However, there has been no report about improvement of growth or improvement of substance productivity under a slow growth rate condition as for the *rmf* gene.

### Disclosure of the Invention

An object of the present invention is to improve production efficiency or production rate in production of a useful substance by fermentation using bacteria belonging to *Escherichia coli*.

The inventors of the present invention assiduously studied in order to achieve the aforementioned object, and as a result, they found a global factor expressed in the stationary phase, and that substance production by bacteria belonging to *Escherichia coli* could be improved by modifying this gene. That is, they identified the *rmf* gene as a gene showing markedly increased expression in the stationary phase of *Escherichia coli* by effectively utilizing a gene expression analysis technique based on the DNA array method (H. Tao, C. Bausch, C. Richmond, F.R. Blattner, T. Conway, *Journal of Bacteriology, 181,* pp.6425-6440, 1999). Further, they also found that disruption of the *rmf* gene improved their growth in the stationary phase and their ability to produce an L-amino acid. Thus, they accomplished the present invention.

That is, the present invention provides the followings.
(1) A method for producing an L-amino acid by utilizing a microorganism comprising the steps of culturing a bacterium belonging to Escherichia coli in a medium to produce and accumulate the L-amino acid in the medium or cells of the bacterium and collecting the L-amino acid, wherein (1) transcription or translation of an rmf gene of the bacterium belonging to Escherichia coli is inhibited, whereby an RMF protein, which is the gene product of the rmf gene, is not produced or the production of the RMF protein is decreased, or (2) an RMF protein is mutated, whereby the original function of the RMF protein is decreased or eliminated.
(2) The method according to (1), wherein the RMF protein does not function normally in the bacterium belonging to *Escherichia coli* due to disruption of *rmf* gene on the chromosome of the bacterium.
(3) The method according to (1) or (2), wherein the L-amino acid is L-lysine.

Hereafter, the present invention will be explained in detail.

The bacterium belonging to the genus *Escherichia* used for the present invention is not particularly limited so long as it is a microorganism that belongs to *Escherichia coli* and has an ability to produce an L-amino acid. Specifically, those mentioned in the work of Neidhardt *et al.* (Neidhardt, F.C. *et al., Escherichia coli* and *Salmonella Typhimurium*, American Society for Microbiology, Washington D.C., 1208, Table 1) can be utilized.

Examples of L-amino acids include L-lysine, L-threonine, L-homoserine, L-glutamic acid, L-leucine, L-isoleucine, L-valine and L-phenylalanine.

As L-lysine producing bacteria belonging to *Escherichia coli*, there can be exemplified mutants having resistance to an L-lysine analogue. The L-lysine analogue inhibits growth of bacteria belonging to the genus *Escherichia,* but this inhibition is fully or partially desensitized when L-lysine coexists in a medium. Examples of the L-lysine analogue include oxalysine, lysine hydroxamate, S-(2-aminoethyl)-L-cysteine (AEC), γ-methyllysine, α-chlorocaprolactam and so forth. Mutants having resistance to these lysine analogues can be obtained by subjecting baceteria belonging to the genus *Escherichia* to a conventional artificial mutagenesis treatment. Specific examples of bacterial strain used for producing L-lysine include *Escherichia coli* AJ11442 (FERM BP-1543, NRRL B-12185; refer to Japanese Patent Laid-open Publication (Kokai) No. 56-18596 and U.S. Patent No. 4,346,170) and *Escherichia coli* VL611. In these microorganisms, feedback inhibition of aspartokinase by L-lysine is desensitized.

In addition to the above, there can be mentioned, for example, L-threonine producing bacteria described later, because inhibition of aspartokinase by L-lysine is generally desensitized also in L-threonine producing bacteria.

In the examples described later, the strain WC196 was used as an L-lysine producing bacterium of *Escherichia coli.* This bacterial strain was bred by conferring AEC resistance to the strain W3110 which was derived from *Escherichia coli* K-12. This strain was designated as the *Escherichia coli* AJ13069 strain, and was deposited at the National Institute of Bioscience and Human-Technology, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on December 6, 1994 and received an accession number of FERM P-14690. Then, it was transferred to an international deposit under the provisions of the Budapest Treaty on September 29, 1995, and received an accession number of FERM BP-5252 (refer to International Patent Publication WO96/17930).

Examples of L-threonine producing bacteria belonging to *Escherichia coli* include *Escherichia coli* VKPM B-3996 (RIA 1867) (refer to U.S. Patent No. 5,175,107), MG442 strain (refer to Gusyatiner *et al., Genetika* (in Russian), *14,* pp.947-956, 1978) and so forth.

Examples of L-homoserine producing bacteria belonging to *Escherichia coli* include the strain NZ10, which is a Leu⁺ revertant of the strain C600 (refer to Appleyard R.K., *Genetics, 39,* pp.440-452, 1954).

Examples of L-glutamic acid producing bacteria belonging to *Escherichia coli* include the AJ12624 strain (FERM BP-3853, refer to French Patent Laid-open Publication No. 2,680,178) and L-valine resistant strains such as *Escherichia coli* B11, *Escherichia coli* K-12 (ATCC10798), *Escherichia coli* B (ATCC11303) and *Escherichia coli* W (ATCC9637).

Examples of L-leucine producing bacteria belonging to *Escherichia coli* include bacterial strains having β-2-thienylalanine resistance, bacterial strains having β-2-thienylalanine resistance and β-hydroxyleucine resistance (refer to Japanese Patent Publication (Kokoku) No. 62-34397 for the above) and bacterial strains having 4-azaleucine resistance or 5,5,5-trifluoroleucine resistance (Japanese Patent Laid-open Publication (Kokai) No. 8-70879). Specifically, there can be mentioned the strain AJ11478 (FERM P-5274, refer to Japanese Patent Publication (Kokoku) No. 62-34397).

Examples of L-isoleucine producing bacteria belonging to *Escherichia coli* include *Escherichia coli* KX141 (VKPM B-4781, refer to European Patent Laid-open Publication No. 519,113).

Examples of L-valine producing bacteria belonging to *Escherichia coli* include *Escherichia coli* VL1970 (VKPM B-4411, refer to European Patent Laid-open Publication No. 519,113).

Examples of L-phenylalanine producing bacteria include *Escherichia coli* AJ12604 (FERM BP-3579, refer to European Patent Laid-open Publication No. 488,424).

Further, bacteria belonging to *Escherichia coli* having L-amino acid producing ability can also be bred by introducing DNA having genetic information involved in biosynthesis of L-amino acids and enhancing the ability utilizing a gene recombination technique. For example, as for L-lysine producing bacteria, examples of genes that can be introduced include, for example, genes coding for enzymes of the biosynthetic pathway of L-lysine such as phosphoenolpyruvate carboxylase, aspartokinase, dihydrodipicolinate synthetase, dihydrodipicolinate reductase, succinyldiaminopimelate transaminase and succinyldiaminopimelate deacylase. In case of a gene of an enzyme suffering from feedback inhibition by L-aspartic acid or L-lysine such as phosphoenolpyruvate carboxylase or aspartokinase and dihydrodipicolinate synthetase, it is desirable to use a mutant gene coding for an enzyme in which such inhibition is desensitized.

Further, as for L-glutamic acid producing bacteria, examples of genes that can be introduced include genes of glutamate dehydrogenase, glutamine synthetase, glutamate synthase, isocitrate dehydrogenase, aconitate hydratase, citrate synthase, phosphoenolpyruvate carboxylase, pyruvate dehydrogenase, pyruvate kinase, phosphoenolpyruvate synthase, enolase, phosphoglyceromutase, phosphoglycerate kinase, glyceraldehyde-3-phosphate dehydrogenase, triose phosphate isomerase, fructose bis-phosphate aldolase, phosphofructokinase, glucose phosphate isomerase and so forth.

As for L-valine producing bacteria, examples of genes that can be introduced include, for example, an *ilvGMEDA* operon, preferably, an *ilvGMEDA* operon that does not express threonine deaminase activity and in which attenuation is cancelled (refer to Japanese Patent Laid-open Publication (Kokai) No. 8-47397).

Further, the activity of an enzyme that catalyzes a reaction for producing a compound other than the target L-amino acid by branching off from the biosynthetic pathway of the L-amino acid may be decreased or made deficient. For example, examples of such an enzyme that catalyzes a reaction for producing a compound other than L-lysine by branching off from the biosynthetic pathway of L-lysine include homoserine dehydrogenase (refer to International Patent Publication WO95/23864). Further, examples of an enzyme that catalyzes a reaction for producing a compound other than L-glutamic acid by branching off from the biosynthetic pathway of L-glutamic acid include α-ketoglutarate dehydrogenase, isocitrate lyase, phosphate acetyltransferase, acetate kinase, acetohydroxy acid synthase, acetolactate synthase, formate acetyltransferase, lactate dehydrogenase, glutamate decarboxylase, 1- pyrroline dehydrogenase and so forth.

Further, bacteria belonging to *Escherichia coli* having an ability to produce a nucleic acid are described in detail in, for example, International Patent Publication WO99/03988. More specifically, there can be mentioned the *Escherichia coli* FADRaddG-8-3::KQ strain (*purFKQ*, *purA*^{*-*}*, deoD*^{*-*}*, purR*^{*-*}*, add*^{*-*}*, gsk*^{*-*}) described in that publication. This strain has ability to produce inosine and guanosine. This strain contains a mutant *purF* gene coding for PRPP amidotransferase in which the lysine residue at a position of 326 is replaced with a glutamine residue and feedback inhibition by AMP and GMP is desensitized, and in this strain, the succinyl AMP synthase gene (*purA*), purine nucleoside phosphorylase gene (*deoD*), purine repressor gene (*purR*), adenosine deaminase gene (*add*) and inosine-guanosine kinase gene (*gsk*) are disrupted. This strain was given a private number of AJ13334, and deposited at the National Institute of Bioscience and Human-Technology, Agency of Industrial Science and Technology, Ministry of International Trade and Industry (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on June 24, 1997 as an international deposit under the provisions of the Budapest Treaty and received an accession number of FERM BP-5993.

In addition, bacteria belonging to *Escherichia coli* having ability to produce other L-amino acids can also be used for the present invention.

In bleeding of bacteria belonging to *Escherichia coli* having such an L-amino acid producing ability as mentioned above, to introduce a gene into *Escherichia* bacteria to enhance their ability, there can be used a method in which a vector autonomously replicable in a cell of bacterium belonging to *Escherichia coli* is ligated to the gene to construct recombinant DNA and *Escherichia coli* is transformed with it. In addition, it is also possible to incorporate a target gene into host chromosome by a method using transduction, transposon (Berg, D.E. and Berg, C.M., *Bio*/*Technol. 1,* p.417, 1983), Mu phage, (Japanese Patent Laid-open Publication (Kokai) No. 2-109985) or homologous recombination (Experiments in Molecular Genetics, Cold Spring Harbor Lab., 1972).

The bacterium belonging to *Escherichia coli* used for the present invention is a bacterium that has such ability to produce an L-amino acid as mentioned above and wherein transcription or translation of the *rmf* gene is inhibited and thus the RMF protein, which is its gene product, is not produced or its production is decreased, or that a mutation is introduced into the produced RMF protein and thus the original function of the RMF protein is decreased or eliminated. Typical examples of the bacterium belonging to *Escherichia coli* in which the RMF protein does not function normally include a gene disrupted strain in which the *rmf* gene on chromosome is disrupted by means of gene recombination techniques, and a mutant in which the functional RMF protein is no longer produced because a mutation is introduced into an expression control sequence of the *rmf* gene or a coding region on chromosome.

Hereafter, an example of the method for disrupting the *rmf* gene on chromosome by means of a gene recombination technique will be explained. The *rmf* gene on chromosome can be disrupted by transforming a bacterium belonging to *Escherichia coli* with DNA containing an *rmf* gene modified so as not to produce RMF functioning normally by deleting a part of the *rmf* gene (deletion type *rmf* gene) and causing recombination between this deletion type *rmf* gene and the *rmf* gene on the chromosome. Such gene disruption by homologous recombination has already been established, and there are methods utilizing linear DNA, a plasmid that contains a temperature sensitive replication control region or the like. In view of reliability, the method utilizing a plasmid that contains a temperature sensitive replication control region is preferred.

The *rmf* gene on the host chromosome can be replaced with the deletion type *rmf* gene as follows. That is, it is possible that recombinant DNA is prepared by inserting a temperature sensitive replication control region, a mutant *rmf* gene and a marker gene showing resistance to a drug such as ampicillin, a bacterium belonging to *Escherichia coli* is transformed with this recombinant DNA, and the transformant strain is cultured at a temperature at which the temperature sensitive replication control region does not function and then further cultured in a medium containing the drug to obtain a transformant strain in which the recombinant DNA is incorporated into the chromosomal DNA.

The strain in which the recombinant DNA is incorporated into the chromosome DNA as described above causes recombination with the *rmf* gene sequence originally existing on the chromosome, and two fusion genes of the chromosome *rmf* gene and the deletion type *rmf* gene are inserted into the chromosome on the both sides of the other part of the recombinant DNA (vector portion, temperature sensitive replication control region and drug resistance marker). Therefore, the transformant strain expresses a normal RMF protein, since the normal *rmf* gene is dominant in this state.

Subsequently, in order to maintain only the deletion type *rmf* gene on the chromosome DNA, one copy of the *rmf* gene is eliminated from the chromosome DNA along with the vector region (including the temperature sensitive replication control region and the drug resistance marker) by recombination of two of the *rmf* genes. At this time, there is a case where the normal *rmf* gene is left on the chromosomal DNA and the deletion type *rmf* gene is eliminated, or a case where, conversely, the deletion type *rmf* gene is left on the chromosomal DNA and the normal *rmf* gene is eliminated. In either case, the removed DNA is harbored in the cell in the form of a plasmid when the strain is cultured at a temperature at which the temperature sensitive replication control region functions. On the other hand, if the strain is cultured at temperature at which the temperature sensitive replication control region does not function, a plasmid containing the normal *rmf* gene is removed from the cell when the deletion type *rmf* gene is left on the chromosome DNA. Therefore, by confirming the structure of the *rmf* gene in the cell by colony PCR or the like, there can be obtained a strain containing the deletion type *rmf* gene on the chromosome DNA, from which cell the normal *rmf* gene is removed.

As a plasmid having a temperature sensitive replication control region that functions in a cell of bacterium belonging to *Escherichia coli*, there can be mentioned pMAN997 (International Patent Publication WO99/03988), which was used in the examples described later.

Techniques used for usual gene recombination such as digestion and ligation of DNA, transformation, extraction of recombinant DNA from a transformant strain and PCR are described in detail in references well known to those skilled in the art, for example, Sambrook, J., Fritsch, E.F., Maniatis, T., Molecular Cloning, Cold Spring Harbor Laboratory Press, 1989 and so forth.

Further, a mutant strain in which the RMF protein having a function is no longer produced can be obtained by treating a bacterium belonging to *Escherichia coli* by ultraviolet radiation or with a mutagenesis agent used for a conventional mutation treatment such as N-methyl-N'-nitro-N-nitrosoguanidine (NTG) or nitrous acid.

An L-amino acid can be produced by culturing a bacterium belonging to *Escherichia coli* obtained as described above, which has an L-amino acid producing ability and of which RMF protein does not function normally in its cell, in a medium to produce and accumulate the L-amino acid in the medium or a cell, and collecting the target substance. In the present invention, the production rate or production efficiency of the L-amino acid can be improved by using a bacterium belonging to *Escherichia coli* having the aforementioned property. It is inferred that this is because, while the *rmf* gene is expressed in the stationary phase of culture and the protein translation activity is decreased in a wild strain of a bacterium belonging to *Escherichia coli* containing the *rmf* gene, decrease of the protein translation activity is prevented or reduced in a strain in which the normal RMF protein does not function normally.

As the medium used for culture of bacteria belonging to *Escherichia coli* in the present invention, conventionally used well known media can be used depending on the kind of the used bacterial strain or the L-amino acid. That is, usual media containing a carbon source, nitrogen source, inorganic ion and other organic components as required can be used. No special medium for carrying out the present invention is required.

As the carbon source, sugars such as glucose, lactose, galactose, fructose and starch hydrolysate, alcohols such as glycerol and sorbitol, organic acids such as fumaric acid, citric acid and succinic acid and so forth can be used.

As the nitrogen source, inorganic ammonium salts such as ammonium sulfate, ammonium chloride and ammonium phosphate, organic nitrogen such as soybean hydrolysate, ammonia gas, aqueous ammonia and so forth can be used.

The organic trace nutrient source preferably contains appropriate amounts of required substances such as Vitamin B₁, L-homoserine and L-tyrosine, yeast extract and so forth. In addition to these, small amounts of potassium phosphate, magnesium sulfate, iron ion, manganese ion and so forth are added as required.

The culture may be performed under well known conditions that are conventionally used depending on the used bacterial strain. For example, culture is preferably performed under an aerobic condition for 16-120 hours. The culture temperature is controlled to be 25-45°C and pH is controlled to be 5-8 during the culture. Inorganic or organic acidic or alkaline substances as well as ammonia gas and so forth can be used for pH adjustment.

To collect a target substance from a medium or cells after the culture is finished, no special method is required for the present invention. That is, collection of the target substance can be attained by a combination of well known methods such as those using an ion exchange resin, precipitation and others depending on the kind of the target substance. Further, the target substance accumulated in cells can be collected from cell extract or membrane fraction depending on the target substance after physically or enzymatically disrupting the cells. Depending on the target substance, the target substance can be utilized as a microbial catalyst or the like while it is existent in cells.

According to the present invention, production rate or production efficiency can be improved in production of L-amino acids by using *Escherichia coli* bacteria.

### Brief Description of the Drawings

Fig. 1 shows growth patterns of the WC196Δrmf strain, which are results of experiments performed with two colonies each at n = 3 for each experimental field. The error bars represent standard errors (the same shall apply to the following figures).
Fig. 2 shows sugar consumption patterns of the WC196Δrmf strain.
Fig. 3 shows lysine accumulation patterns of the WC196Δrmf strain.
Fig. 4 shows growth of the WC196Δrmf/pMPI700 strain obtained by introducing an acid phosphatase gene into the WC196Δrmf strain and a control strain.
Fig. 5 shows staining intensities of bands of acid phosphatase observed in SDS polyacrylamide gel electrophoresis of crude enzyme solutions of the WC196Δrmf/pMPI700 strain and the control strain.

### Best Mode for Carrying out the Invention

Hereafter, the present invention will be explained more specifically with reference to the following examples.

### Example 1: Extraction of total RNA from Escherichia coli cell and analysis by using DNA macroarray

A wild strain of *Escherichia coli*, the strain W3110, was cultured at 37°C in 300 ml of medium containing 16 g/L of MS medium (20 g/L of glucose, 1 g/L of KH₂PO₄, 1 g/L of MgSO₄, 2 g/L of yeast extract, (NH₃)₂SO₄) by using a 1-L volume small-size jar fermenter. The culture was performed with aeration at a flow rate of 150 ml/min, while the stirring number was controlled so that the dissolved oxygen concentration should become 5% or more. Further, the pH of the medium was adjusted to be constant at 6.8 by feeding aqueous ammonia, and when glucose in the medium was depleted, 500 g/L glucose solution was fed so that the glucose concentration in the culture broth should be in the range of 0.1-10 g/L.

Separately, the strain W3110 was cultured at 37°C with reciprocal shaking at 120 rpm by using a 500-ml volume Sakaguchi flask with 50 ml of medium containing E-100 medium (20 mM NH₄Cl, 2 mM MgSO₄, 40 mM NaHPO₄, 30 mM KH₂PO₄, 0.01 mM CaCl₂, 0.01 mM FeSO₄, 0.01 mM MnSO₄, 5 mM citric acid, 50 mM glucose, 2 mM thiamine hydrochloride, 2.5 g/L of casamino acid (Difco) and 250 mM MES-NaOH (pH 6.8)).

To extract total RNA from *Escherichia coli* cells, about 1 ml and about 10 ml of culture broths were sampled from the jar fermenter and the flask respectively, during the logarithmic growth phase and the stationary phase of the growth curve. Each sampled culture broth was immediately cooled on ice and centrifuged at 10,000 g for 2 minutes by a cooled centrifugation machine, and the culture broth supernatant was discarded. The total RNA was collected from the collected cells by using an RNeasy Kit produced by QIAGEN according to the attached protocol. It was confirmed by agarose gel electrophoresis that the obtained total RNA was collected without being decomposed, and absorption at 260 nm was measured to quantify the RNA concentration. The obtained total RNA was sealed and stored at -80°C, and then used for gene expression analysis using DNA macroarrays.

A reverse transcription reaction was performed with 20 µg of the obtained total RNA as a template, 1 mM each of dATP, dGTP and dTTP and 9.25 MBq of [α-³²P] dCTP as substrate by using a Reverse Transcription Kit produced by Promega to obtain labeled cDNA in the logarithmic growth phase and the stationary phase.

Hybridization was performed by using the obtained cDNA as a probe and a Panorama *E*. *coli* Gene Arrays macroarray membrane produced by Sigma-Genosys according to the attached protocol. When the hybridization was finished, the membrane was washed. The washed membrane was sealed and closely contacted with an imaging plate produced by Fuji Photo Film for 48 hours for exposure. The exposed imaging plate was read by a fluoroimaging analyzer FLA-3000G produced by Fuji Photo Film. Density at each spot in the obtained read image was determined by using a DNA array image analysis system, AIS (Imaging Research), and converted into an expression ratio to obtain the gene expression profile data in the logarithmic growth phase and the stationary phase.

According to the obtained DNA array data, a group of genes of which expression level was low in the logarithmic growth phase, but increased in the stationary phase was selected from the total genes of *Escherichia coli.* The ratio of the expression ratio in the stationary phase to the expression ratio in the logarithmic growth phase was calculated for respective genes obtained from the jar fermenter and the flask culture. There were extracted genes that showed the 20 highest values for a value obtained by multiplying increasing ratios in the expression ratios of the two types of cultures. Among these, the *rmf* gene was extracted as the one showing the highest value among genes of which function was known. Changes in the growth rate and the expression rate of the *rmf* gene are shown in Table 1. It was revealed that it was a gene for which a marked increase in expression was observed in the stationary phase for both of the cultures in jar fermenter and flask.

**Table 1: Relationship between growth rate and rmf gene expression amount in each culture condition and culture phase**

| Medium | Culture vessel | Culture time (hr) | Specific growth ratio (1/hr) | *rmf* expression ratio* |
|---|---|---|---|---|
| MS | Jar | 1.5 | 0.32 | 1.000 |
| MS | Jar | 9 | 0.02 | 11.068 |
| E-100 | Flask | 4 | 0.75 | 1.063 |
| E-100 | Flask | 24 | -0.019 | 47.711 |

| | | | | |
|---|---|---|---|---|
| *: Relative value with respect to the value obtained for the jar culture for 1.5 hours, which is taken 1 | | | | |

### Example 2: Disruption of rmf gene of Escherichia coli and effect on L-lysine production

The *rmf* gene of *Escherichia coli* was disrupted by crossover PCR (refer to Link, A.J., Phillips, D., Church, G.M., *J. Bacteriol.*, *179*, pp.6228-6237, 1997).

The oligonucleotides of SEQ ID NOS: 1 and 2 (Primers 1 and 2) were synthesized as primers for amplifying a region of about 1 kbp including about 600 bp of a coding region of the *rmf* gene for the N-terminus and a region upstream therefrom, and the oligonucleotides of SEQ ID NOS: 3 and 4 (Primers 3 and 4) were synthesized as primers for amplifying a region of about 1 kbp including about 600 bp of a coding region of the *rmf* gene for the C-terminus and a region downstream therefrom. Primers 2 and 3 were designed to have complementary common sequences as parts thereof so that a part of the *rmf* gene ORF should be lost when amplified products were ligated at this portion.

A first PCR was performed by using combinations of Primers 1 and 2 and Primers 3 and 4 and genomic DNA of a wild strain prepared by a conventional method, the W3110 strain, as a template. At this time, the mole ratio of Primers 1 and 2 and Primers 4 and 3 was 10:1. A second PCR was performed by using the obtained product of the first PCR as a template and Primers 1 and 4. A DNA fragment having a deficient type *rmf* gene constructed by the second PCR was cloned into a cloning vector kit, pGEMT-easy produced by Promega, according to its protocol to obtain a recombinant vector pGEM-R.

pGEMdR was digested with *Eco*RI to obtain a DNA fragment containing the deficient type *rmf* gene. This digested fragment and a temperature sensitive plasmid pMAN997 digested with the same enzyme and purified (refer to International Patent Publication WO99/03988) were ligated by using a DNA Ligation Kit Ver.2 (Takara Shuzo). The aforementioned pMAN997 was obtained by exchanging the *Vsp*I*-Hin*dIII fragment of pMAN031 (*J*. *Bacteriol., 162*, p.1196, 1985) and that of pUC19 (Takara Shuzo).

*Escherichia coli* JM109 competent cells (Takara Shuzo) were transformed with the above ligation reaction mixture, seeded on an LB agar plate containing 25 µg/ml of ampicillin (Meiji Seika, LB + ampicillin) and cultured at 30°C to select an ampicillin resistant colony. The colony was cultured at 30°C in a test tube with LB medium containing 25 µg/ml of ampicillin, and plasmids were extracted from cells by using Wizard Plus Miniprep (Promega). These plasmids were digested with EcoRI, and a plasmid containing a target length fragment was used as a plasmid for *rmf* disruption, pMANΔrmf.

The *Escherichia coli* WC196 strain was transformed with pMANΔrmf. The WC196 strain is an AEC resistant L-lysine producing *Escherichia coli* bacterium, and it was given a private number of AJ13069 and deposited at the National Institute of Bioscience and Human-Technology, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan), and received an accession number of FERM P-14690. Then, it was transferred to an international deposit under the provisions of the Budapest Treaty on September 29, 1995, and received an accession number of FERM BP-5252 (refer to International Patent Publication WO96/17930).

The transformant strain was cultured at 30°C on an LB + ampicillin plate, and an ampicillin resistant colony was selected. The selected colony was liquid-cultured overnight at 30°C, diluted 10⁻³-fold and seeded on an LB + ampicillin plate, and an ampicillin resistant colony was selected at 42°C. At this stage, pMANΔrmf was incorporated into chromosomal DNA.

Subsequently, the selected colony was spread over an LB + ampicillin plate and cultured at 30°C, and then an appropriate amount of cells were suspended in 2 ml of LB medium and cultured at 42°C for 4-5 hours with shaking. Ampicillin susceptibility or resistance was confirmed by seeding a 10⁻⁵-fold diluted culture broth on an LB plate, seeding several hundreds of colonies among the obtained colonies on an LB plate and an LB + ampicillin plate and confirming their growth. In chromosome DNA of ampicillin susceptible strains, there were removed a vector portion of pMANΔrmf and a normal *rmf* gene originally existent on the chromosome DNA or a deletion type *rmf* gene. Several ampicillin susceptible strains were subjected to colony PCR to select a strain wherein the *rmf* gene was replaced with a deletion type gene as intended. Thus, an *rmf* gene disrupted strain, the WC196Δrmf strain, was obtained from the L-lysine producing *Escherichia coli* bacterium, WC196.

The *rmf* gene disrupted strain, the WC196Δrmf strain, and its parent strain, the WC196 strain, were cultured in a medium containing 20 mM NH₄Cl, 2 mM MgSO₄, 40 mM NaHPO₄, 30 mM KH₂PO₄, 0.01 mM CaCl₂, 0.01 mM FeSO₄, 0.01 mM MnSO₄, 5 mM citric acid, 50 mM glucose, 2 mM thiamine hydrochloride, 2.5 g/L casamino acid (Difco) and 250 mM MES-NaOH (pH 6.8) by using a 200-ml volume baffled conical flask. The culture broth was used in an amount of 20 ml at the start of the culture and culture was performed at 37°C with rotary shaking at a rotational speed of 144 rpm. The medium, container and so forth were all subjected to autoclave sterilization before use.

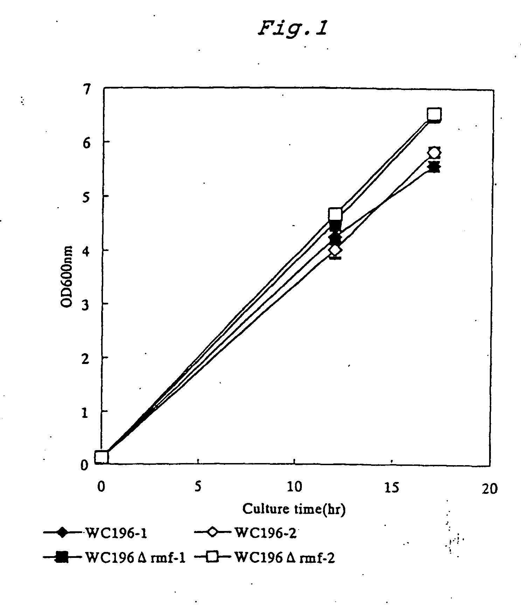

The cell density, glucose concentration and L-lysine accumulation in the culture broth were measured in the time course. The cell density was obtained by measuring turbidity at 600 nm with a spectrophotometer (Beckman) by using the culture broth diluted with water to an appropriate density. The culture broth supernatant sterilized by centrifugation was diluted to an appropriate concentration with water, and then the glucose concentration and the L-lysine concentration were measured by using Biotech Analyzer (Sakura Seiki). The results are shown in Figs. 1 to 3. Values of L-lysine accumulation and remaining sugar after 17 hours of culture are also shown.

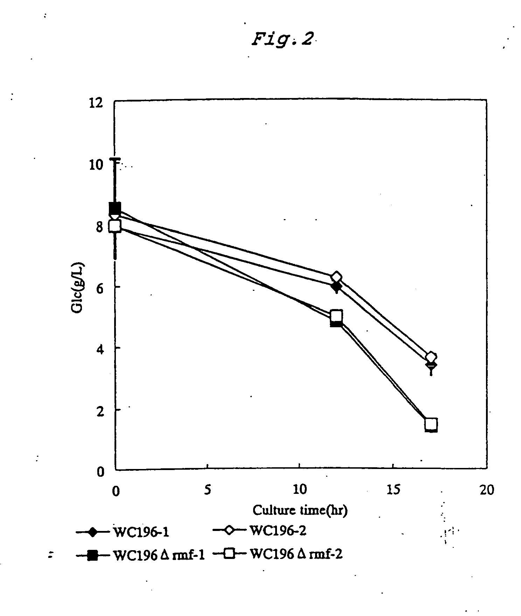

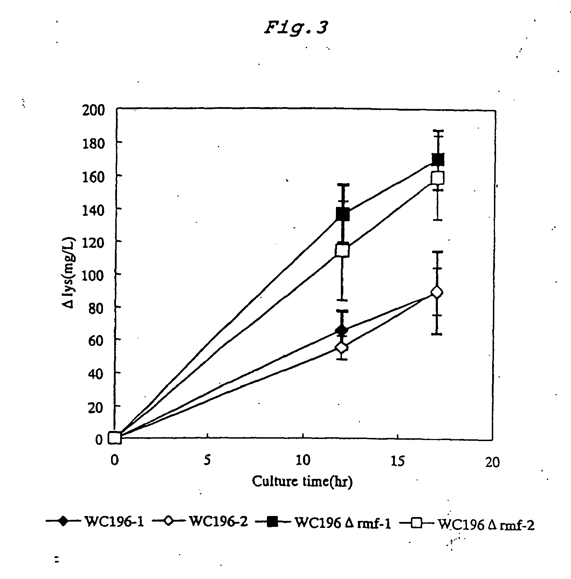

As a result, it was recognized that the *rmf* gene disrupted strain was improved in all of growth (Fig. 1), sugar consumption rate (Fig. 2) and L-lysine production rate (Fig. 3), compared with the control strain.

**Table 2: L-lysine production of WC196Δrmf strain**

| Bacterial strain | Experiment | Cultur e time (hr) | L-lysine accumulation (as hydrochloride) (mg/L) | Remaining sugar (g/L) |
|---|---|---|---|---|
| WC196 | 1 | 17 | 89 | 3.4 |
| | 2 | 17 | 90 | 3.7 |
| WC196Δrmf | 1 | 17 | 170 | 1.4 |
| | 2 | 17 | 159 | 1.5 |

### Example 3 (not belonging to the scope of the invention): Effect of Disruption of rmf gene of Escherichia coli on protein production

### (1) Introduction of acid phosphatase overexpressing plasmid into rmf gene disrupted strain and expression thereof

The *rmf* gene disrupted strain obtained in Example 2, the WC196Δrmf strain, and its parent strain, the WC196 strain, were transformed with a plasmid pMPI700 containing a mutant type acid phosphatase gene to obtain WC196/pMPI700 strain and WC196Δrmf/pMPI700 strain. The mutant type acid phosphatase is a mutant enzyme having a decreased 5'-nucleotidase activity (phosphate ester hydrolysis activity) while maintaining a nucleoside 5'-phosphate ester producing activity (phosphotransfer activity), and its 92nd glycine residue and 171st isoleucine residue are replaced with an aspartic acid residue and a threonine residue, respectively. pMPI700 is a plasmid obtained as follows (*Applied and Environmental Microbiology, 66* (7), pp.2811-2816, July 2000).

By using the plasmid pMPI501 containing an acid phosphatase gene derived from *Morganella morganii* NCIMB 10466 (Japanese Patent Laid-open Publication (Kokai) No. 9-37785; U.S. Patent No. 6,010,851) as a template, the gene was introduced with a random mutation by error-prone PCR. The acid phosphatase gene fragment (*Eco*RI-*Hin*dIII) introduced with a mutation was introduced into pUC18, and *E. coli* JM109 was transformed with the obtained recombinant plasmid. One transformant strain having phosphotransfer activity equivalent to that of *E. coli* (pMPI501) and decreased 5'-nucleotidase activity was obtained among the transformants and designated as JM109 (pMPI600). The random mutation was similarly introduced again by using the plasmid pMPI600 contained in this strain to obtain the plasmid pMPI700.

An *Escherichia coli* strain JM109 harboring a plasmid pMPI505 containing a DNA fragment obtained by further shortening the acid phosphatase gene fragment contained in pMPI501 by subcloning was designated as AJ13143. This strain has been deposited at the National Institute of Bioscience and Human-Technology, Agency of Industrial Science and Technology, Ministry of International Trade and Industry (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan), which is an international depository under the provisions of the Budapest Treaty, on February 23, 1996 and received an accession number of FERM BP-5422. A plasmid similar to pMPI700 can be obtained by using pMPI505 instead of pMPI501.

The strains WC196/pMPI700 and the WC196Δrmf/pMPI700 were cultured in LB medium containing 100 g/ml of ampicillin by using a 500-mL volume Sakaguchi flask. The culture broth was used in an amount of 50 ml at the start of the culture and culture was performed at 37°C with reciprocal shaking at a rotational speed of 120 rpm. The medium, container and so forth were all subjected to autoclave sterilization before use. At this time, the cell density in the culture broth was measured. The cell density was obtained by measuring turbidity at 600 nm with a spectrophotometer (Beckman) by using the culture broth diluted with water to an appropriate density. Further, a culture broth containing cells was collected in a time course and cells were collected by centrifugation, suspended in 100 mM phosphate buffer (pH 7.0), disrupted by ultrasonication for 20 minutes and centrifuged again to obtain a supernatant as a crude enzyme solution, which was used for measurements of protein concentration and acid phosphatase enzyme activity. The protein concentration in the crude enzyme solution was measured by the Bradford method.

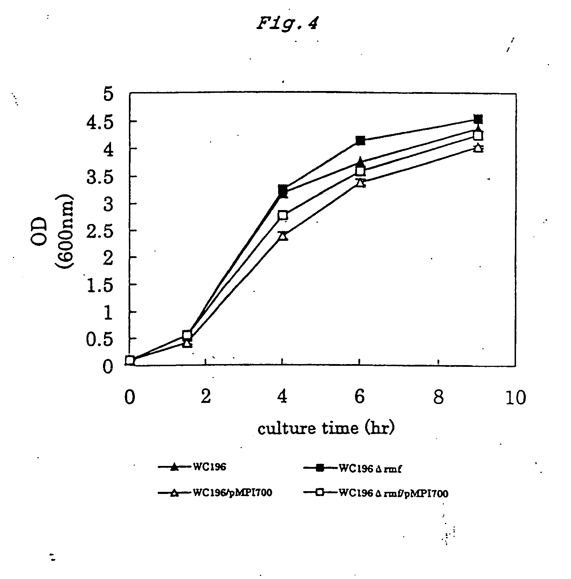

The enzyme activity was measured as follows. An enzymatic reaction was performed at 30°C in a 100 mM MES/KOH buffer (pH 6.0) by using 10 mM p-nitrophenyl phosphate as a substrate. One minute after the addition of the crude enzyme solution, the reaction mixture was added with 1/5 volume of 2 N KOH to terminate the reaction. After centrifugation, *p*-nitrophenol phosphate produced in the supernatant was quantified. The produced *p*-nitrophenol phosphate was quantified by measuring absorption at 410 nm using a spectrophotometer (Beckman). Further, the enzyme activity was calculated by assuming the molar absorption coefficient of *p-*nitrophenol phosphate as 17.52 mM⁻¹·cm⁻¹. The results are shown in Table 3. Growth of each bacterial strain during the culture (OD₆₀₀) is shown in Fig. 4.

As a result, it was recognized that the *rmf* gene disrupted strain was improved in all of growth, specific activity and total activity per unit volume of culture broth, compared with the control strains (Table 3)

**Table 3: Acid phosphatase activity of crude enzyme solution**

| Bacterial strain | Culture time (hr) | Specific activity/mg of protein | Total activity (U/ml) |
|---|---|---|---|
| WC196 | 6 | 0.071 | 0.019 |
| WC196Δrmf | 6 | 0.045 | 0.054 |
| WC196/pMPI700 | 6 | 2.02 | 0.569 |
| WC196Δrmf/pMPI700 | 6 | 4.08 | 1.190 |

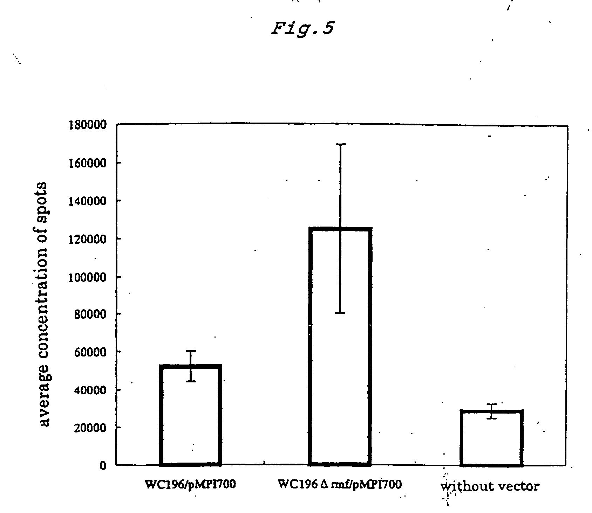

Further, the above crude enzyme solution was subjected to SDS gel electrophoresis using 15% polyacrylamide gel and stained with CYPRO Orange (Bio-Rad), and the stained gel image was read by a fluoroimaging analyzer FLA-3000G produced by Fuji Photo Film. The concentration at each spot was quantified from the obtained read image by using the image analysis software, Image Gauge, to confirm expression of the target enzyme protein and compare expression amounts. As a result, a target band was detected, and its concentration well reflected the specific activity (Fig. 5).

## Claims

1. A method for producing an L-amino acid by utilizing a microorganism comprising the steps of culturing a bacterium belonging to *Escherichia coli* in a medium to produce and accumulate the L-amino acid in the medium or cells of the bacterium and collecting the L-amino acid, wherein (1) transcription or translation of an *rmf* gene of the bacterium belonging to *Escherichia coli* is inhibited, whereby an RMF protein, which is the gene product of the *rmf* gene, is not produced or the production of the RMF protein is decreased, or (2) an RMF protein is mutated, whereby the original function of the RMF protein is decreased or eliminated.

2. The method according to claim 1, wherein the RMF protein does not function normally in the bacterium belonging to *Escherichia coli* due to disruption of *rmf* gene on the chromosome of the bacterium.

3. The method according to claim 1 or 2, wherein the L-amino acid is L-lysine.

## Patentansprüche

1. Verfahren zur Produktion einer L-Aminosäure durch Verwendung eines Mikroorganismus, welches die Stufen umfasst, bei denen ein zu Escherichia coli gehörendes Bakterium in einem Medium kultiviert wird, wobei die L-Aminosäure in dem Medium oder den Zellen des Bakteriums hergestellt und angehäuft wird, und die L-Aminosäure gewonnen wird, wobei (1) die Transkription oder Translation eines rmf-Gens des zu Escherichia coli gehörenden Bakteriums inhibiert ist, wodurch ein RMF-Protein, welches das Genprodukt des rmf-Gens ist, nicht produziert wird oder die Produktion des RMF-Proteins verringert ist, oder (2) ein RMF-Protein mutiert ist, wodurch die ursprüngliche Funktion des RMF-Proteins beeinträchtigt oder ausgeschaltet ist.

2. Verfahren nach Anspruch 1, wobei das RMF-Protein in dem zu Escherichia coli gehörenden Bakterium aufgrund der Störung des rmf-Gens auf dem Chromosom des Bakteriums nicht normal funktioniert.

3. Verfahren nach Anspruch 1 oder 2, wobei die L-Aminosäure L-Lysin ist.

## Revendications

1. Méthode pour la production d'un L-aminoacide par utilisation d'un microorganisme, comprenant les étapes de culture d'une bactérie appartenant à *Escherichia coli* dans un milieu pour produire et accumuler le L-aminoacide dans le milieu ou des cellules de la bactérie, et de recueil de l'aminoacide, dans laquelle (1) la transcription ou la traduction d'un gène *rmf* de la bactérie appartenant à *Escherichia coli* est inhibée, si bien qu'une protéine RMF, qui est le produit du gène *rmf,* n'est pas produite, ou que la production de la protéine RMF est diminuée, ou (2) une protéine RMF est mutée, si bien que la fonction d'origine de la protéine RMF est diminuée ou éliminée.

2. Méthode selon la revendication 1, dans laquelle la protéine RMF ne fonctionne pas normalement dans la bactérie appartenant à *Escherichia coli* du fait d'une perturbation du gène *rmf* sur le chromosome de la bactérie.

3. Méthode selon la revendication 1 ou 2, dans laquelle le L-aminoacide est la L-lysine.
